# EUROPEAN PATENT APPLICATION

(11) **EP 3 181 701 A1**
(43) Date of publication of application: **21.06.2017**
(21) Application number: 15382635.9
(22) Date of filing: 17.12.2015
(51) Int. Cl.: C12Q 1/68

(54) **METHOD FOR THE SUB-CLASSIFICATION OF PATIENTS SUFFERING FROM PARKINSON DISEASE**

(71) Applicant: CONSEJO SUPERIOR DE INVESTIGACIONES CIENTIFICAS, 28006 Madrid (ES); Centro de Investigación Biomédica en Red de Enfermedads Neurodegenerativas (CIBERNED), 41012 Sevilla (ES); Fundacio Clinic Per A La Recerca Biomedica, 08036 Barcelona (ES); Hospital Clínic de Barcelona, 08036 Barcelona (ES); Universitat de Barcelona, 08028 Barcelona (ES)
(72) Inventor: TRULLAS OLIVA, RAMON, 28006 Madrid (ES); PODLESNIY, Petar, 41012 Sevilla (ES); VILAS ROLÁN, Dolores, 08036 Barcelona (ES); TOLOSA SARRÓ, Eduardo, 08036 Barcelona (ES)
(74) Representative: Pons

(57) **Abstract**

The invention relates to the use of mitochondrial DNA as a quantitative biomarker for the sub-classification of patients suffering from Parkinson disease in the subgroups of familial or idiopathic Parkinson. The invention also relates to a method and to a kit for the sub-classification of patients suffering from Parkinson disease using said biomarker.

## Description

This invention belongs to the field of methods for the diagnosis and sub-classification of patients suffering from neurodegenerative diseases based on the quantification of biomarkers. Specifically, it refers to methods that allow the sub-classification of Parkinson's patients in the idiopathic and familial or genetic form of the disease.

### BACKGROUND

Mutations in the *LRRK2* gene are the most common known cause of inherited Parkinson Disease (PD) and account for a significant proportion of idiopathic cases. Six different mutations of the *LRRK2* gene produce autosomal dominant PD and the most common is the missense mutation that modifies glycine to serine in the amino acid 2019 (G2019S) at the kinase domain 1. *LRRK2-PD* and idiopathic PD (IPD) are clinically indistinguishable although some differences have been reported in *LRRK2*-PD cohorts, such as a more benign course, less olfactory impairment and a better cognitive profile. The *LRRK2*^{G2019S} mutation has a reduced age-dependent penetrance, but the mechanisms whereby a significant percentage of asymptomatic LRRK2 mutation carriers never develop PD are still unknown.

The majority of the genes involved in familial PD, including *LRRK2,* are directly or indirectly involved in mitochondrial function or dynamics (Ryan, BJ, et al., 2015, Trends Biochem Sci., 40:200-210). Particularly, *LRRK2* can be found in the mitochondrial outer membrane. Studies in cortical cultured neurons have shown that overexpression of wild type *LRRK2* inhibits mitochondrial fusion and enhances mitochondrial fission and the latter effect is strengthened by the G2019S mutation. Likewise, the physiological expression of *LRRK2*^{G2019S} mutation in knock-in mice markedly alters mitochondrial morphology and reduces basal dopamine release in the striatum before neurodegeneration, mimicking the pre-clinical changes in dopaminergic neurotransmission that occur in human PD.

The majority of PD cases are idiopathic with an unknown cause and no pathophysiological biomarkers have been identified yet that allow a precise diagnosis during lifetime at any disease stage. It is generally assumed that the pathogenic mechanisms involved in familial PD may help to understand the pathophysiology of the idiopathic form of the disease. Actually, like in the case of monogenic forms of hereditary PD, there is extensive evidence showing that mitochondrial function is also altered in idiopathic PD (Johri, A and Beal, MF., 2012, J Pharmacol Exp Ther., 342:619-630). However, whether the genetic and idiopathic forms share similar mechanisms of mitochondrial dysfunction is unclear.

Mitochondrial DNA (mtDNA) is a key regulator of mitochondrial function. It has been disclosed in a previous publication that there is a low concentration of circulating cell free mtDNA in cerebrospinal fluid (CSF) in asymptomatic familial and sporadic forms of Alzheimer's disease (AD), suggesting that mitochondrial dysfunction precedes biomarkers of neuronal damage in AD (WO2014080054 A1).

Despite diagnostic methods have been described for determining the presence or absence of PD in a subject in which the levels of mtDNA are measured as a biomarker (Angela, Pyle, et al., 2015, ANN NEUROL., doi: 10.1002/ana.24515), there is a need to develop diagnostic methods for PD that additionally allow the sub-classification of patients suffering from the disease in subgroups corresponding to the different forms of the same, specifically in the idiopathic and familial or genetic form. Identification of clinical biomarkers of PD subgroups is increasingly important to develop neuroprotective strategies. Since familial and IPD forms of the disease present pathophysiological differences they may require different treatments, thus these sub-classification methods would allow the selection of the most suitable therapy to be administered to each patient depending on the form of the disease. These methods could also have a significant impact for the development of new drugs for the treatment of Parkinson.

### DETAILED DESCRIPTION OF THE INVENTION

This invention proposes the use of mitochondrial DNA (mtDNA) as a quantitative biomarker for the *in vitro* sub-classification of patients suffering from Parkinson disease (PD), and therefore provides a method for said sub-classification based on the use of this biomarker.

To assess whether idiopathic (IPD) and familial forms of PD exhibit a similar mtDNA profile in cerebrospinal fluid (CSF), the inventors of the present invention studied the concentration of cell free mtDNA in CSF from IPD patients and *LRRK2*^{G2019S} mutation carriers, both asymptomatic and with manifest PD. In addition, they investigated the relationship between mtDNA and other CSF biomarkers. These studies have allowed come to the conclusion that *LRRK2-*PD is associated with high content of CSF mtDNA suggesting that different mechanisms of mitochondrial function may underlie idiopathic vs LRRK2-PD. Thus, mtDNA is proposed herein as a pathophysiological biomarker of the disease in familial PD, preferably in *LRRK2*^{G2019S} mutation carriers.

Therefore, high CSF content of mtDNA is a biomarker of disease penetrance in familial or genetic PD, preferably in LRRK2^{G2019S} mutation carriers, and differentiates idiopathic PD from familial PD, preferably LRRK2 related PD. These findings also suggest that familial and idiopathic PD are mediated by different biochemical processes and may require treatments with contrary effects on regulation of mtDNA, which have a significant impact for the development of new drugs.

Therefore, the method described in this invention allows the sub-classification of patients suffering from the disease in its different forms, specifically in the idiopathic and familial form. This method has the advantage that it allows the selection of the most efficient therapeutic treatment to be administered to each patient depending on the form of the disease.

Furthermore, this method can be carried out in a wide variety of biological samples without a reduction in reliability and reproducibility, including without limitation, blood, serum or plasma of the subject under study. Thus, the method of the invention does not necessarily require an invasive procedure in the patient. Furthermore, this procedure is simple to perform, because the quantification of mitochondrial DNA can be performed through techniques widely used for nucleic acid quantification such as, for example but without limitation, PCR. The method described herein is sensitive, specific, reproducible between laboratories and with low variability.

Thus, this invention represents a solution to the need of providing a method for the efficient sub-classification of Parkinson's patients in the familial and the idiopathic forms of the disease, and it can be performed *in vitro* in a biological sample isolated from the patient.

Therefore, a first aspect of this invention refers to the use of mitochondrial DNA, preferably the quantified levels of mitochondrial DNA, as a quantitative biomarker for the *in vitro* sub-classification of patients suffering from Parkinson disease.

The term "sub-classification" is considered to be the procedure through which a subject suffering from Parkinson disease, thus belonging to the group of Parkinson's patients, is assigned to a subgroup of a specific Parkinson form, preferably to the subgroup of familial or genetic Parkinson or the subgroup of idiopathic Parkinson (IPD).

The term "quantitative biomarker", as used in this invention, refers to the amount or concentration of mtDNA used as an indicator useful for the sub-classification of patients suffering from Parkinson disease in familial Parkinson or IPD.

The term "mitochondrial DNA" or "mtDNA" in the present invention refers to the genetic material of mitochondria that can be found both intracellularly and extracellularly or circulating or cell free. The intracellular genetic material is found in the cell and the extracellular or circulating genetic material is found outside the cell such as, but without limitation, in the cerebrospinal fluid, blood, serum, blood, plasma, saliva, urine, tears or sweat. Therefore, in a preferred embodiment, mtDNA referred to in this invention is extracellular mtDNA or circulating or cell free mtDNA.

In a more preferred embodiment, the region of mtDNA that is amplified and quantified in this invention is at least mtDNA-85, in humans. This "mtDNA-85" region corresponds to bases 14,848 to 14,932 of the human mtDNA Cambridge reference sequence (RefSeq: NC_012920.1).

The term "Parkinson disease" or "PD", as used in this invention refers to a progressive disorder of the nervous system that affects movement. Although symptoms and signs may vary with the stages of the disease and from person to person, some of them are, but without limitation, tremor, slowed movement (bradykinesia), rigid muscles, impaired posture and balance, loss of automatic movements and speech and writing changes. Clinical characteristics of PD disease in a subject are preferably assessed according to the Movement Disorder Society-sponsored revision of the Unified Parkinson's disease Rating Scale (MDS-UPDRS) (Goetz, CG., et al., 2008, Mov Disord., 23:2129-2170).

A subject suffering from "idiopathic Parkinson" or "IPD" is a Parkinson patient who does not present genetic mutations in none of the genes associated with familial Parkinson. Genes associated with familial Parkinson are, but not limited to, alpha-synuclein SNCA (*PARK1-4*), PINK1 (*PARK6*), Parkin (*PARK2*), DJ-1 (*PARK7*), ATP13A2 (*PARK9*) or the *LRRK2* (*PARK8*) gene.

A subject suffering from "familial Parkinson" is a Parkinson patient who carries one or more genetic mutations in at least one gene associated to Parkinson disease, such as *LRRK2.* When the patient carries a mutation in *LRRK2* gene it is called *"LRRK2* related Parkinson" patient.

In a second aspect, this invention refers to a method for the *in vitro* sub-classification of patients suffering from Parkinson disease, hereinafter "the method of the invention", comprising the following steps:
a) quantifying mtDNA in a biological sample isolated from a subject suffering from Parkinson,
b) comparing the value obtained in step (a) to a standard value obtained from the quantification of mtDNA in a biological sample isolated from a subject suffering from idiopathic Parkinson, and
c) assigning the subject of step (a) to the group of patients suffering from familial Parkinson when the value obtained in step (a) is significantly higher than the standard value.

Alternatively, steps (b) and (c) of the method of the invention comprise comparing the value obtained in step (a) to a standard value obtained from the quantification of mtDNA in a biological sample isolated from a subject suffering from familial Parkinson, and assigning the subject of step (a) to the group of patients suffering from idiopathic Parkinson when the value obtained in step (a) is significantly lower than the standard value.

In a preferred embodiment, the mtDNA referred to in the method of the invention is mtDNA-85 as described above. In a more preferred embodiment, the mtDNA referred to in the method of the invention is extracellular mtDNA.

In a more preferred embodiment, the method of the invention further comprises the following steps:
d) quantifying alpha-synuclein levels in the biological sample of step (a),
e) comparing the value obtained in step (d) to a standard value obtained from the quantification of alpha-synuclein levels in a biological sample isolated from a subject suffering from idiopathic Parkinson, and
f) assigning the subject of step (a) to the group of patients suffering from familial Parkinson when the value obtained in step (d) is significantly higher than the standard value.

Alternatively, steps (e) and (f) of the method of the invention comprise comparing the value obtained in step (d) to a standard value obtained from the quantification of alpha-synuclein levels in a biological sample isolated from a subject suffering from familial Parkinson, and assigning the subject of step (a) to the group of patients suffering from idiopathic Parkinson when the value obtained in step (d) is significantly lower than the standard value.

In an even more preferred embodiment the familial Parkinson as used in the present invention is LRRK2 related Parkinson. In a particular embodiment, the LRRK2 related Parkinson is due to the presence of the LRRK2^{G2019S} mutation in the subject. *I*. e., in the present invention, the subject suffering from familial Parkinson is preferably a Parkinson patient carring the G2019S mutation in the *LRRK2* gene. These patients or subjects will be also called "LRRK2-PD patients".

The expression "to quantify" the mtDNA or alpha-synuclein levels in an isolated biological sample, as used in this invention, refers to the measurement of the amount or the concentration. Measurement refers to the measurement of the amount or concentration of mtDNA or alpha-synuclein, both intracellular and extracellular, preferably extracellular in the case of mtDNA. Techniques for measuring mtDNA are, for example but without limitation, PCR, quantitative real time PCR (qPCR), digital PCR (dPCR) or *Southern Blot.* Preferably, the amount quantified in this invention is expressed as the number of copies of mtDNA per volume of sample. Techniques for measuring alpha-synuclein levels comprise techniques for the measurement of mRNA or protein, preferably protein. These techniques are, for example but without limitation, PCR, RTLCR, RT-PCR, qRT-PCR, or any other method for the amplification of nucleic acids; DNA microarrays made with oligonucleotides deposited by any technique, DNA microarrays made with *in situ* synthesized oligonucleotides, *in situ* hybridization using specific labeled probes, electrophoresis gels, membrane transfer and hybridization with a specific probe, RMN or any other image diagnosis technique using paramagnetic nanoparticles or other type of functionalized nanoparticles, assays such as Western blot, immunoprecipitation assays, protein arrays preferably antibodies based miroarrays, immunofluorescence, immunohistochemistry, ELISA or any other enzymatic method, by incubation with a specific ligand, chromatographic techniques preferably combined with mass spectrometry. For the immunoassay the antibodies used may be labeled with, for instance, antibodies, an enzyme, radioisotopes, magnetic tags or fluorescence.

In a preferred embodiment, step (a) of the method of the invention is carried out by PCR, qPCR, dPCR or *Southern Blot.* More preferably, step (a) is carried out by qPCR or dPCR. Even more preferably, step (a) is carried out by dPCR.

In a more preferred embodiment, step (d) of the method of the invention is carried out by immunocytochemistry, even more preferably by ELISA.

The term "PCR" in this invention is understood to be the polymerase chain reaction. It is a molecular biology technique where the objective is to obtain a large number of copies of a particular fragment of DNA, starting from the minimum. This technique is based on the natural property of DNA polymerases to replicate DNA strands starting from primers that are linked at the ends of the strands; this is done by using cycles of alternating high and low temperatures to separate the recently formed DNA strands after each phase of replication and joining the primer and then binding the polymerases again in order to duplicate the strands. In this invention, PCR is used for amplification and subsequent quantification of mtDNA in the biological sample, preferably for amplifying the mtDNA-85 sequence. There are various types of PCR such as real time PCR or quantitative PCR and digital PCR (dPCR). The main characteristic of qPCR is that it allows quantifying the amount of DNA in an original sample or identifying, with very high probability, samples of specific DNA from their melting temperature. dPCR is an improvement of the conventional polymerase chain reaction that may be used to directly quantify the nucleic acid content at a level of resolution of a single molecule and to amplify ("clonally amplify") nucleic acids such as DNA, cDNA and RNA. In this invention, the two variants of PCR can be used for quantifying mtDNA, preferably extracellular, in a biological sample.

The term "*Southern Blot*", as used in this invention refers to a molecular biology method that enables detecting the presence of a DNA sequence in a complex mixture of these nucleic acids and quantifying the DNA present in a biological sample. To do this, the technique of electrophoresis in agarose gel is used in order to separate the DNA fragments by length and, later, transfer them to a membrane in which hybridisation with a probe is performed.

The term "amount" as used in the description refers, but without limitation, to the absolute or relative amount of mtDNA or alpha-synuclein, preferably expressed as the number of copies of mtDNA or alpha-synuclein, as well as any other value or parameter related to this or that can be derived from this. These values or parameters comprise values of signal intensity obtained from any of the physical or chemical properties of mtDNA or alpha-synuclein obtained by direct measurement, for example, values of intensity of mass spectroscopy or nuclear magnetic resonance. Additionally, these values or parameters include all those obtained through indirect measurement such as production of proteins coded for by the mtDNA, generation of free radicals or production of energy.

The term "isolated biological sample" as used in this description refers to a sample isolated from an organism that might come from a physiological fluid and/or any cell or tissue of the organism. In a preferred embodiment of the method of the invention, the isolated biological sample is selected from the list consisting of: saliva, sweat, tears, urine, cerebrospinal fluid, blood, serum and blood plasma. In a more preferred embodiment, the isolated biological sample is cerebrospinal fluid. In another preferred embodiment, the biological sample is blood, serum or blood plasma. In a more preferred embodiment, the biological sample is serum.

The term "cerebrospinal fluid" as used in this description refers to a liquid of transparent colour that bathes the brain and spinal cord. Many diseases change its composition and its study is important and frequently determining, for the diseases of the central and peripheral nervous system. Included in these diseases are the neurodegenerative diseases such as Alzheimer's or Parkinson's disease.

The term "standard value" is considered to mean any value or range of values derived from the quantification of mtDNA or alpha-synuclein levels in a biological sample from an individual as explained above or in a mixture of biological samples derived from said individuals.

The group of individuals chosen for obtaining the standard value have the same or similar age as the subject under study and are representative of the population in which the method of the invention is to be applied. The quantification must be made in the same way and be obtained from the same type of isolated biological sample as that from the subject to be studied in step (a) of the method of the invention.

The term "comparison" as used in this invention refers, but is not limited to, the comparison of the amount of mtDNA and alpha-synuclein determined in the biological sample of step (a) with a standard value. The comparison described in steps (b) and (e) of the method of the invention can be performed manually or computer-assisted.

An amount which is "significantly higher" than a standard value can be established by an expert in the field through the use of various statistical tools such as, for example but without limitation, by determination of confidence intervals, determination of the p value, two-tailed unpaired Student's t test, Fisher's discriminant functions, using Kruskal-Wallis one way analysis with Dunn's multiple comparisons test, one-way ANOVA with Bonferroni's post hoc multiple comparisons test, two tailed Mann-Whitney U test or Kruskal-Wallis U tests.

The subject referred to in step (a) of the method of the invention may be a human, but also a non-human mammal such as, for example but without limitation, rodents, ruminants, felines or dogs. Therefore, in another preferred embodiment, the subject from which the biological sample of step (a) of the method of the invention is isolated is a mammal. In a more preferred embodiment, the mammal is a human.

In a third aspect, this invention refers to a kit for the *in vitro* sub-classification of patients suffering from Parkinson disease, hereinafter "the kit of the invention", that comprises:
- a probe comprising the structure 6-carboxyfluorescein (FAM)-SEQ ID NO: 1-Black Hole Quencher-1 (BHQ-1),
- a forward primer of SEQ ID NO: 2, and
- a reverse primer of SEQ ID NO: 3.

In a fourth aspect, this invention refers to the use of a kit comprising specific primers and/or probes for mtDNA for the *in vitro* sub-classification of patients suffering from Parkinson, wherein the kit preferably further comprises specific antibodies for alpha-synuclein.

In a preferred embodiment, these specific primers and/or probes for mtDNA are specific for mtDNA-85 as described above. In a more preferred embodiment of the fourth aspect of the invention, this kit is the kit of the invention described above.

The kit of the invention may comprise, without limitation, labelled or unlabelled primers, labelled or unlabelled probes, buffers, agents for preventing contamination, marker compounds such as, for example but without limitation, fluorophores, etc. The kit of the invention may also include all the supports and recipients necessary for implementing and optimising it. The kit of the invention may contain positive and negative controls. Preferably, this kit also comprises instructions to carry out the quantification of mtDNA, and preferably the quantification of alpha-synuclein, according to the description given in this invention.

In a preferred embodiment, the set of primers or probes of the kit of the invention is labelled or immobilised. In a more preferred embodiment, the set of primers or probes of the kit of the invention is labelled with a label selected from the list comprising: a radioisotope, fluorescent or luminescent marker, antibody, antibody fragment, affinity label, enzyme and enzyme substrate. In a yet more preferred embodiment, the set of primers or probes of the kit of the invention is immobilised on a support such as, for example but without limitation, in a nylon matrix, plastic support or beads.

In this description, the term "primer" is a nucleic acid chain that serves as a starting point for the amplification of DNA. It is a short nucleic acid sequence containing a free 3' hydroxyl group that forms complementary base pairs to the template strand and acts as a starting point for the addition of nucleotides in order to copy and amplify the template strand. In this invention, the primers are bound to the ends of the mtDNA for amplification and detection by, for example but without limitation, PCR.

The term "probe" as used in this description refers to a small DNA fragment labelled with fluorescence that emits signals detectable by laser and is used as a tool in, for example but without limitation, qPCR or dPCR to quantify mtDNA in a biological sample.

Specific primers and/or probes for mtDNA, preferably for mtDNA-85, can be designed by techniques for designing primers and/or probes that are well known in the state of the art. Preferably, the primers referred to in this invention are those of SEQ ID NO: 2 and SEQ ID NO: 3. Preferably, the probe this invention refers to is 6-carboxyfluorescein (FAM)-SEQ ID NO: 1-Black Hole Quencher-1 (BHQ-1).

Throughout the description and claims, the word "comprise" and its variants does not exclude other technical characteristics, additives, components or steps. For experts in the field, other objects, advantages and characteristics of the invention will emerge, partly from the description and partly from the practice of the invention. The following examples and figures are provided for the purpose of illustration only and are not intended to limit this invention.

### DESCRIPTION OF THE FIGURES

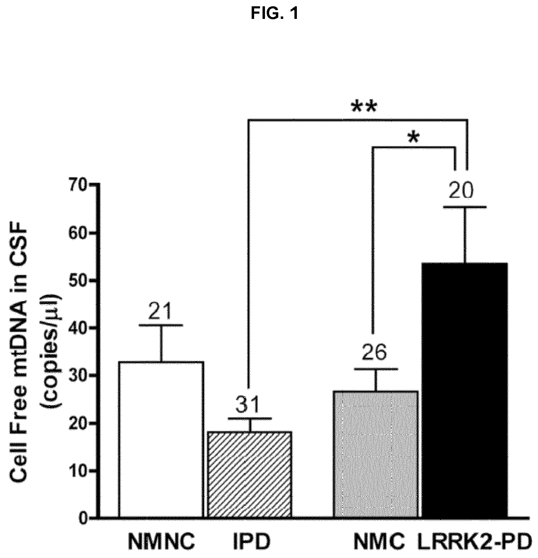

**Fig. 1****. High cerebrospinal fluid mitochondrial DNA content in LRRK2, but not in idiopathic, Parkinson's disease.** Cell-free circulating mtDNA copy number was measured directly in CSF samples by droplet digital PCR. NMNC (non-manifesting non-carriers): control subjects who do not carry a mutation in the *LRRK2* gene and do not manifest PD at the time of enrollment. IPD, subjects who have a confirmed clinical diagnosis of idiopathic PD but do not carry a LRRK2 mutation. NMC (non-manifesting carriers), subjects who carry a G20192 mutation in the *LRRK2* gene and do not manifest PD. LRRK2-PD, subjects who carry a mutation in the *LRRK2* gene and have PD. Values are mean±SEM. Numbers above error bars indicate the number of subjects per group. *, p<0.05; **, p<0.01 (two tailed Mann-Whitney U test).

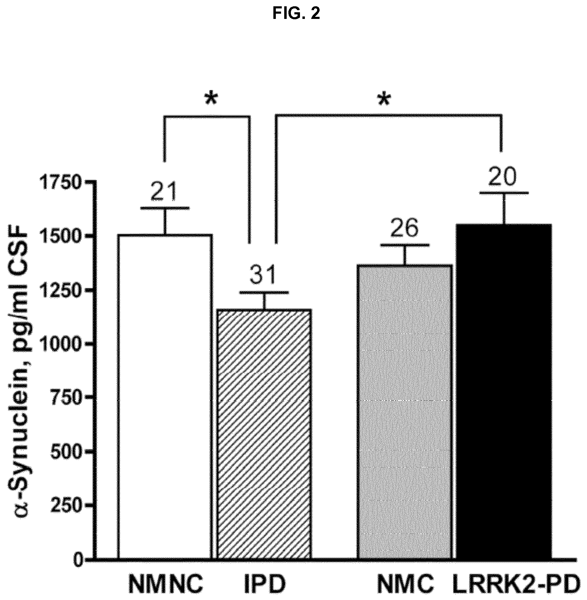

**Fig. 2****. Low cerebrospinal fluid content of α-Synuclein in idiopathic, but not in LRRK2-related Parkinson's disease.** NMNC (non-manifesting non-carriers): control subjects who do not carry a mutation in the *LRRK2* gene and do not manifest PD at the time of enrollment. IPD, subjects who have a confirmed clinical diagnosis of idiopathic PD but do not carry a LRRK2 mutation. NMC (non-manifesting carriers), subjects who carry a G20192 mutation in the *LRRK2* gene and do not manifest PD. LRRK2-PD, subjects who carry a mutation in the *LRRK2* gene and have PD. Values are mean±SEM. Numbers above error bars indicate the number of subjects per group.*, p<0.05; (two tailed Mann-Whitney U test).

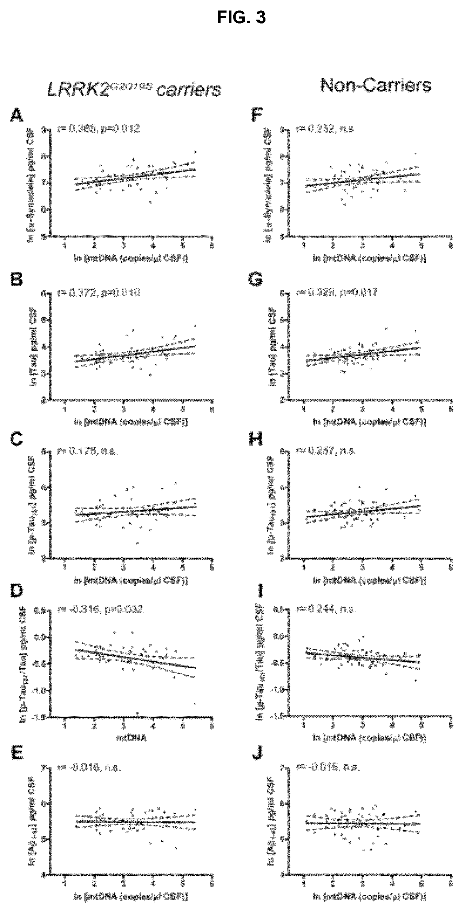

**Fig. 3****. Relationship between cell-free mtDNA concentration and biomarker profile in CSF from LRRK2^{G2019S} mutation carriers and Non-Carriers.** Linear regression graphs and Pearson correlation (r) analysis between mtDNA concentration and Amyloid β1-42, t-tau, ratio of p-tau₁₈₁/t-tau, α-synuclein levels in CSF (n.s.= non-significant). The natural logarithm (In) of values was used for regression analyses.

### EXAMPLES

The invention is illustrated below with some tests performed by the inventors that demonstrate the efficacy of the use of mtDNA as a quantitative biomarker for the sub-classification of patients suffering from Parkinson disease. These specific examples are provided to illustrate the nature of this invention and are only included for illustrative purposes; they must not be interpreted as limiting the invention claimed here. Therefore, the examples described below illustrate the invention without limiting its field of application.

### Example 1. Determination of cell free mitochondrial DNA (mtDNA) content in cerebrospinal fluid (CSF) from LRRK2 mutation carriers and idiopathic Parkinson's disease (IPD) patients.

The concentration of mtDNA was measured using digital droplet PCR in a total of 98 CSF samples from a cohort of subjects including: 20 *LRRK2*^{G2019S} mutation carriers with Parkinson's disease (LRRK2-PD), 26 asymptomatic *LRRK2*^{G2019S} mutation carriers (non-manifesting carriers, NMC), 31 patients with IPD and 21 first-degree relatives of LRRK2-PD patients without the mutation (non-manifesting non carriers, NMNC).

As it will be shown below, LRRK2-PD patients exhibit high concentration of mtDNA in CSF compared with NMC and with IPD patients. In addition, IPD, but not LRRK2-PD, is associated with low CSF concentration of alpha-synuclein. Thus, high mtDNA content in CSF distinguishes LRRK2-PD from IPD suggesting that different biochemical pathways underlie neurodegeneration in these two disorders. Moreover, these results suggest that mtDNA plays a key role in the pathophysiology of LRRK2-PD disease.

### 1.1. Subjects and Methods.

### Subjects.

The study cohort consisted of CSF samples from a total of 98 subjects from the Michael J. Fox Foundation (MJFF) *LRRK2* Cohort Consortium. Subjects were classified based on clinical diagnosis and genetic profile in four different categories: 1) subjects who carry the G2019S mutation in the *LRRK2* gene and have PD (*LRRK2-PD*), n=20; 2) subjects who carry the G2019S mutation in the *LRRK2* gene and do not manifest PD (non-manifesting carriers = NMC), n=26; 3) subjects who have a confirmed clinical diagnosis of PD but do not carry the *LRRK2*^{G2019S} mutation (IPD), n= 31 and 4) subjects who do not carry the *LRRK2*^{G2019S} mutation and do not manifest PD at the time of enrollment (non-manifesting non-carriers; NMNC), n=21. The latter group was comprised primarily of non-carrying first degree relatives of patients. All participants were assessed for clinical characteristics based on the Movement Disorder Society-sponsored revision of the Unified Parkinson's disease Rating Scale (MDS-UPDRS) (Goetz, CG, Tilley, BC, Shaftman, SR, et al., 2008, Mov Disord., 23:2129-2170).

### CSF sample collection and handling.

CSF collection was performed following the protocol detailed in the Parkinson's progression markers initiative (PPMI) biologics manual as previously described (Kang, JH, et al., 2013, JAMA Neurol., 70:1277-1287). Frozen aliquots of CSF in siliconized polypropylene tubes were received from the *LRRK2* Cohort Consortium biorepository Core Laboratories on dry ice and stored at -80°C until the day of the assay. Samples were coded and analyses were performed in a blind manner. Each assay included samples from the four different patient groups that were counterbalanced before samples were coded by a scientist from the Biorepository Core Laboratory.

### Droplet digital PCR.

The CSF concentration of cell free circulating mtDNA CSF was measured by droplet digital PCR in a Bio-Rad QX200 Digital Droplet PCR platform (Bio-Rad Laboratories, Hercules, CA 94547, USA). The PCR reaction was performed with a hydrolysis probe and primers that produce an amplicon of 85 base pairs (mtDNA-85).

Reactions were made in three replicates and assayed in 96 well plates. The sequence of the mtDNA probe is 6-carboxyfluorescein (FAM)-5'-CCTCCAAATCACCACAGGACTATTCCTAGCCATGCA-3' (SEQ ID NO: 1)-Black Hole Quencher-1 (BHQ-1). The sequences of the mtDNA primers are: Forward CTCACTCCTTGGCGCCTGCC (SEQ ID NO: 2) and Reverse GGCGGTTGAGGCGTCTGGTG (SEQ ID NO: 3). These primers bind an mtDNA target sequence that is not associated with known single polymorphisms and do not have a full complementary target in the human genome based on the information available at the National Institutes of Health (USA) genetic sequence database GenBank at the time of assay. The PCR amplicon obtained with these mtDNA primers corresponds to bases 14,848-14,932 of the human mtDNA Cambridge reference sequence NC_012920.1.

PCR assay conditions were adjusted to obtain a single amplicon analyzed by both melting curve analysis and agarose gel electrophoresis.

To determine the presence of contaminating cells in the CSF, mtDNA and genomic DNA were simultaneously measured in the CSF multiplex dPCR assay using the mtDNA primers described above in addition to a hydrolysis probe and primers to amplify the single copy nuclear gene BAX. The sequence of the BAX probe is: 6 - carboxy - 2',4,4',5',7,7' - hexachlorofluorescein HEX-CCCGAGCTGGCCCTGGACCCGGT (SEQ ID NO: 4)-BHQ1. Primer sequences are: Forward, TTCATCCAGGATCGAGCAGG (SEQ ID NO: 5) and Reverse, TGAGACACTCGCTCAGCTTC (SEQ ID NO: 6). These primers amplify a single amplicon of 103 base pairs corresponding to apoptosis regulator BAX isoform alpha (BAX-103). CSF samples contaminated with cells (>1 copy of BAX per microliter of CSF) were excluded from the study. Characterization experiments with either single or multiplex assays of mtDNA-85 and BAX-103 using the procedures described above yield the same results. The dPCR assay was performed in a 20 µl reaction volume consisting of 1X ddPCRSupermix for probes (BioRad186-3023), 900 nM forward and reverse primers, 100 nM mtDNA FAM labeled probe, 300 nM BAX HEX labeled probe and 4.50 µl of unprocessed CSF sample. A volume of double-distilled water equivalent to CSF sample was used as non-template control (NTC). Two or more non template controls (NTCs) were included in each ddPCR assay plate. Droplet emulsion formation was performed by mixing the 20 µl reaction with 70 µl droplet generation oil using a microfluidic droplet generation cartridge and QX200 Droplet Generator. End point PCR amplifications were performed using C1000 Thermal Cycler with the following conditions: 95°C 10min; 40 repeats of 94°C 30sec; 60°C 1 min; 98°C 10min. The presence or absence of amplification per droplet was evaluated using QX200 Droplet Reader and analyzed using QuantaSoft protocol. The results were expressed in copies of mtDNA per µl of CSF.

### Analysis of CSF biomarkers.

CSF amyloid beta (Aβ1-42), total tau (T-tau) and phospho-tau (p-tau₁₈₁) were measured using the xMAP-Luminex platform with INNOBIA AlzBio3 immunoassay kit-based reagents (Fujirebio-Europe, Ghent, Belgium), as previously described (Kang, JH, et al., 2013, JAMA Neurol., 70:1277-1287). CSF alpha-synuclein (α-syn) was analyzed using an ELISA assay available commercially from BioLegend (cat # 844101) following the method described previously (Kang, JH, et al., 2013, JAMA Neurol., 70:1277-1287).

### Statistical Analysis.

Results are expressed as mean ± SEM. For linear regression analyses, values were transformed to natural logarithm. Statistical significance was examined with GraphPad Prism software using Kruskal-Wallis one way analysis of variance with Dunn's multiple comparison tests or with one-way ANOVA with Bonferroni post hoc multiple comparisons depending on whether data passed normality tests. Comparison between two groups was performed with two tailed Mann-Whitney U tests or with two-tailed unpaired Student's t tests.

### 1.2. Results.

Demographic, clinical and neurodegeneration related biomarker data.

Demographic, clinical and biomarker data are showed in Table 1. CSF was obtained by lumbar puncture for diagnostic purposes. n= number of subjects. Values are mean ±SEM. *, p<0.05, significantly different from NMC, (Oneway ANOVA with Bonferroni post-hoc comparisons):

**Table 1. Characteristics of patient groups.**

| GROUP Characteristic | NMNC (n=21) | IPD (n=31) | NMC (n=26) | LRRK2-PD (n=20) |
|---|---|---|---|---|
| Age (years) | 54±3 | 58±2 | 52±3 | 64±3* |
| Female/male, n (% male) | 10/11 (52%) | 10/21 (68%) | 10/16 (62%) | 11/9 (45%) |
| Disease duration (years) | - | 4.06±0.65 | - | 7.37±1.18 |
| MDS-UPDRS-III score | - | 29.85±2.04 | - | 23.36±2.57 |
| t-tau [pg/ml] | 46±4 | 40±3 | 42±3 | 48±5 |
| p-tau [pg/ml] | 30±2 | 27±2 | 29±2 | 30±3 |
| p-tau/t-tau | 0.673±0.02 | 0.686±0.02 | 0.720±0.03 | 0.670±0.03 |
| Aβ₁₋₄₂[pg/ml] | 262±16 | 231±12 | 253±10 | 248±16 |

As it has been already said, the study included CSF samples from a total of 98 subjects distributed in four groups depending on the presence or absence of *LRRK2*^{G2019S} mutation and clinical diagnosis of PD. LRRK2-PD patients have a significantly higher mean age (64±3 years) than the NMC group (52±3 years) (p<0.05), but do not differ significantly from IPD or NMNC groups (p>0.05) (Oneway ANOVA with Bonferroni post-hoc comparisons). Disease duration and MDS-UPDRS III total scores are not significantly different between LRRK2-PD and IPD patients. In addition, there are no significant differences in CSF t-tau, p-tau, ratio of p-tau/t-tau or Aβ1-42 levels between groups.

### Cell-free mtDNA content in CSF.

Cell free mtDNA concentrations in CSF from *LRRK2* mutation carriers and non-carriers with or without PD are shown in Fig. 1. LRRK2-PD patients show a significant increase in CSF content of mtDNA compared with NMC (54±12 copies/µl, n=20 vs 27±5 copies/µl, n=26, respectively) (p<0.05). Likewise, LRRK2-PD patients have significantly higher CSF mtDNA compared with IPD patients (54±12 copies/µl, n=20 vs 18±3, n=31, respectively) (p<0.01). In contrast, IPD patients exhibit a small but statistically non-significant decrease in the CSF concentration of mtDNA compared to NMNC (18±3 copies/µl, n=31 vs 33±8 copies/µl, n=21, respectively) (p>0.05, two-tailed Mann-Whitney U tests).

### α-synuclein levels in CSF.

Fig. 2 shows α-synuclein levels in CSF from *LRRK2* mutation carriers and non-carriers with or without PD. The CSF content of α-synuclein is significantly lower in the IPD group (1159±51 pg/ml, n=31) compared to the NMNC and *LRRK2-*PD groups (1501±126 pg/ml, n=21 and 1550±151 pg/ml, n=20, respectively) (p<0.05), but it is not significantly different from the NMC group (1364±93 pg/ml, n=26) (p>0.05, two-tailed Mann-Whitney U tests).

Relationship between CSF mtDNA, α-synuclein and neurodegeneration related biomarkers in LRRK2^{G2019S} carriers and non-carriers.

The relationship between CSF concentrations of mtDNA with α-synuclein and other biomarkers associated with neurodegeneration is represented in Fig. 3. Regression analyses in LRRK2^{G2019S} mutation carriers indicate that mtDNA content in CSF correlates positively with CSF levels of α-synuclein (r=0.365, p=0.012, n=46, Fig. 3A), total levels of tau protein (t-tau) (r=0.372, p=0.010, n=46, Fig. 3B) and, negatively, with the ratio of p-tau₁₈₁/tau (r=-0.316, p=0.032, n=46 Fig. 3D). In contrast, in non-carriers, CSF mtDNA levels only correlate significantly with CSF levels of t-tau (r=0.329, p=0.017, n=52, Fig .3G).

The finding that high CSF mtDNA concentration discriminates *LRRK2*^{G2019S} mutation carriers that manifest PD from asymptomatic mutation carriers is consistent with converging lines of evidence suggesting that mitochondria play a key role in the pathogenesis of *LRRK2*-PD and supports the hypothesis that an increase in the CSF level of mtDNA is a pathophysiological biomarker of LRRK2-related PD. Several studies in patient-derived tissue have reported that the *LRRK2*^{G2019S} mutation is associated with mitochondrial dysfunction. However, mitochondrial dysfunction occurs in *LRRK2*^{G2019S} mutation carriers independently of whether they manifest PD or not. The present results showing that high mtDNA content in CSF occurs only in LRRK2^{G2019S} mutation carriers that manifest PD strongly suggest that CSF mtDNA is linked with disease pathophysiology in these patients.

The presence of *LRRK2*^{G2019S} mutation alone is not sufficient to alter the content of mtDNA in CSF since the CSF content of mtDNA in NMC is undistinguishable from the observed in NMNC, implying that other factors act in conjunction with the *LRRK2* mutation to increase CSF mtDNA content in LRRK2-PD patients. This suggests that, in addition to mitochondrial dysfunction, other mechanisms related with regulation of mtDNA copy number that cause an increase in CSF mtDNA content are involved in disease penetrance in LRRK2^{G2019S} mutation carriers. It is also possible that mtDNA content in CSF increases over the time in NMC, meaning that those NMC with normal mtDNA levels are still far away from the onset of motor symptoms. Future longitudinal studies in this cohort of subjects at risk of PD will help to clarify whether high CSF mtDNA is a prognostic biomarker of LRRK2 related parkinsonism.

The CSF content of mtDNA is markedly higher, by approximately 50%, in LRRK2-PD patients than in NMC. On the contrary, CSF mtDNA levels in patients with IPD tend to be lower than in NMNC, although this effect did not reach statistical significance. Given the opposed regulation of CSF mtDNA found in these two groups of PD patients, this data support the hypothesis that mtDNA content in CSF is a biomarker that distinguishes idiopathic from LRRK2 related PD. Moreover, the fact that high mtDNA content in CSF occurs in *LRRK2-PD,* but not in IPD patients, suggests that certain biochemical processes that underlie LRRK2-PD are different from those that mediate the idiopathic form of the disease. In agreement with this interpretation, it has been found herein a significant decrease of α-synuclein CSF concentration in IPD, but not in LRRK2-PD patients. In addition, a positive relationship between CSF mtDNA and α-synuclein levels in LRRK2^{G2019S} mutation carriers has been found, while in non-carriers this relationship is not significant, suggesting that these two groups have a different biomarker profile and providing further evidence that different molecular pathways underlie the sporadic and genetic forms of PD.

The source of cell-free mtDNA in the CSF is currently unknown. Previous evidence suggests that the level of mtDNA in CSF is not associated with cell damage but that it is related with neuronal mtDNA copy number. One possibility is that mtDNA is released from cells into CSF in a physiological process of mitochondrial quality control known as trans-mitophagy that targets mitochondria for trans-cellular mitochondrial degradation. Studies in cultured neurons and neuroblastoma cells have shown that the *LRRK2*^{G2019S} mutation disrupts mitochondrial dynamics and enhances mitophagy. The alteration of any of these pathways of mitochondrial quality control might be responsible for the increase in mtDNA found in the CSF of *LRRK2-PD.*

The contrasting CSF mtDNA levels found in genetic versus idiopathic PD also indicate that the amount of mtDNA present in CSF is not the result of neuronal damage caused by the disease. Rather, mtDNA content in CSF probably reflects a fundamental biochemical process that precedes neurodegeneration. Consistent with this notion, it has been previously shown that low CSF mtDNA is an early biomarker that precedes both sporadic and familial forms of Alzheimer's disease. The discovery that CSF mtDNA content is high in LRRK2-PD, but low in Alzheimer's disease, suggests that opposite regulation of mtDNA replication or degradation underlies these neurodegenerative diseases and reveals that changes in CSF mtDNA content are not the product of neurodegeneration.

In summary, the present results suggest that high CSF content of mtDNA is a biomarker of disease penetrance in LRRK2^{G2019S} mutation carriers and differentiates idiopathic PD from LRRK2 related PD. These findings also suggest that familial and idiopathic PD are mediated by different biochemical processes and may require treatments with contrary effects on regulation of mtDNA, which could have a significant impact for the development of new drugs.

## Claims

1. Use of mitochondrial DNA as a quantitative biomarker for the *in vitro* sub-classification of patients suffering from Parkinson disease.

2. Use according to claim 1, wherein the mitochondrial DNA is extracellular.

3. A method for the *in vitro* sub-classification of patients suffering from Parkinson disease comprising the following steps:
a) quantifying mitochondrial DNA in a biological sample isolated from a subject suffering from Parkinson,
b) comparing the value obtained in step (a) to a standard value obtained from the quantification of mitochondrial DNA in a biological sample isolated from a subject suffering from idiopathic Parkinson, and
c) assigning the subject of step (a) to the group of patients suffering from familial Parkinson when the value obtained in step (a) is significantly higher than the standard value.

4. The method according to claim 3, further comprising the following steps:
d) quantifying alpha-synuclein levels in the biological sample of step (a),
e) comparing the value obtained in step (d) to a standard value obtained from the quantification of alpha-synuclein levels in a biological sample isolated from a subject suffering from idiopathic Parkinson, and
f) assigning the subject of step (a) to the group of patients suffering from familial Parkinson when the value obtained in step (d) is significantly higher than the standard value.

5. The method according to any of claims 3 or 4, wherein the familial Parkinson is LRRK2 related Parkinson.

6. The method according to claim 5, wherein the LRRK2 related Parkinson is due to the presence of the LRRK2^{G2019S} mutation in the subject.

7. The method according to any of claims 3 to 6, wherein the isolated biological sample is selected from the list consisting of: saliva, sweat, tears, urine, cerebrospinal fluid, blood, serum and blood plasma.

8. The method according to claim 7, wherein the isolated biological sample is cerebrospinal fluid.

9. The method according to any of claims 3 to 8, wherein the step (a) is carried out by PCR, quantitative PCR, digital PCR or *Southern blot,* preferably by digital PCR.

10. The method according to any of claims 4 to 9, wherein the step (d) is carried out by immunocytochemistry, preferably by ELISA.

11. The method according to any of claims 3 to 10, wherein the mitochondrial DNA is extracellular.

12. The method according to any of claims 3 to 11, wherein the subject is a human.

13. Kit for the *in vitro* sub-classification of patients suffering from Parkinson disease that comprises:
- a probe comprising the structure 6-carboxyfluorescein (FAM)-SEQ ID NO: 1-Black Hole Quencher-1 (BHQ-1),
- a forward primer of SEQ ID NO: 2, and
- a reverse primer of SEQ ID NO: 3.

14. Use of a kit comprising specific primers and/or probes for mitochondrial DNA for the *in vitro* sub-classification of patients suffering from Parkinson, wherein the kit preferably further comprises specific antibodies for alpha-synuclein.

15. Use according to claim 14, wherein the kit is the kit according to claim 13.


SEQUENCE LISTING

<110> Consejo Superior de Investigaciones Científicas
      Fundació Clínic per a la Recerca Biomèdica
      Hospital Clínic de Barcelona
      Universitat de Barcelona
      Centro de Investigación Biomédica en Red de Enfermedades
      Neurodegenerativas

<120> METHOD FOR THE SUB-CLASSIFICATION OF PATIENTS SUFFERING FROM
      PARKINSON DISEASE

<130> EP1641.1191

<160> 6

<170> PatentIn version 3.5

<210> 1
<211> 36
<212> DNA
<213> Artificial Sequence

<220>
<223> mtDNA probe

<400> 1
cctccaaatc accacaggac tattcctagc catgca                                36


<210> 2
<211> 20
<212> DNA
<213> Artificial Sequence

<220>
<223> mtDNA Forward primer

<400> 2
ctcactcctt ggcgcctgcc                                                  20


<210> 3
<211> 20
<212> DNA
<213> Artificial Sequence

<220>
<223> mtDNA Reverse primer

<400> 3
ggcggttgag gcgtctggtg                                                  20


<210> 4
<211> 23
<212> DNA
<213> Artificial Sequence

<220>
<223> BAX probe

<400> 4

cccgagctgg ccctggaccc ggt                                                23

<210>  5
<211>  20
<212>  DNA
<213>  Artificial Sequence

<220>
<223>  BAX Forward primer

<400>  5
ttcatccagg atcgagcagg                                                     20

<210>  6
<211>  20
<212>  DNA
<213>  Artificial Sequence

<220>
<223>  BAX Reverse primer

<400>  6
tgagacactc gctcagcttc                                                     20